# EUROPEAN PATENT SPECIFICATION

(11) **EP 1 408 944 B1**
(45) Date of publication and mention of the grant of the patent: **25.05.2005**
(21) Application number: 02741579.3
(22) Date of filing: 19.06.2002
(51) Int. Cl.: A61K 31/16, A61P 1/12, A61P 1/00, A61P 25/00

(54) **ERUCAMIDE COMPOUNDS FOR THE TREATMENT AND PREVENTION OF DISTURBANCES OF THE SECRETORY SYSTEM**
ERUCAMID-VERBINDUNGEN ZUR BEHANDLUNG UND PRÄVENTION VON STÖRUNGEN DES SEKRETORISCHEN SYSTEMS
COMPOSES DERIVES DE L'ERUCAMIDE DESTINES AU TRAITEMENT ET A LA PREVENTION DES DESORDRES DU SYSTEME SECRETEUR

(30) Priority: 27.06.2001 SE 0102289
(43) Date of publication of application: 21.04.2004
(73) Proprietor: Hamberger, Anders, 439 31 Onsala (SE); Stenhagen, Gunnar, 431 36 Mölndal (SE)
(72) Inventor: Hamberger, Anders, 439 31 Onsala (SE); Stenhagen, Gunnar, 431 36 Mölndal (SE)
(74) Representative: Bergstrand, Mikael Gudmundsson
(86) International application number: PCT/SE2002/001197
(87) International publication number: WO 2003/002112

(56) References cited:
- WO-A1-96/09817
- WO-A1-99/55159
- WO-A2-98/11887
- WO-A2-99/26584
- US-A- 5 612 380
- US-A- 5 952 392
- CRAVATT BENJAMIN F. ET AL.: 'Chemical characterization of a family of brain lipids that induce sleep' SCIENCE vol. 268, June 1995, pages 1506 - 1509, XP002102114
- BEZUGLOV V.V. ET AL.: 'Bioactive amides of fatty acids' BIOCHEMISTRY vol. 63, no. 1, 1998, MOSCOW, pages 27 - 37, XP002954540
- MURILLO-RODRIGUEZ ERIC ET AL.: 'Oleamide modulates memory in rats' NEUROSCIENCE LETTERS vol. 313, 2001, pages 61 - 64, XP002954539

## Description

### TECHNICAL FIELD

The present invention relates to a novel use for a molecule in the treatment of diseases caused by disturbances and/or defects in the secretion system for fluids in the body, and to formulations containing this compound.

### BACKGROUND OF THE INVENTION

A number of conditions, leading to disease, are linked to an imbalance of secretion and uptake of fluids by cells in various organs. These conditions include an excess of extracellular fluid volume and sometimes inflammation, in e.g. intestine (diarrhea), brain (cerebral edema, hydrocephalus), inner ear (Menière's disease) and eye (glaucoma), rhinitis and hypersalivation, but also generalized in the body (certain types of hypertension).

Infectious diarrhea is a prominent cause of death in many developing countries and diarrhea has a great impact on children also in the industrialised world. The disease consists of intestinal fluid loss, which can cause dehydration and death within hours after the onset. The prototype for organisms causing diarrhea by means of an enterotoxin is *Vibrio cholerae*. Diarrhea-causing enterotoxins are also produced by *E. coli,* Salmonella and *Clostridium difficile*.

Brain edema (swelling) is a potential complication of trauma, ischemia, hemorrhage, tumors and other conditions affecting the brain. The critical parameter for the survival of the patient with brain edema is the intracranial pressure (ICP), which may increase to levels which abolish oxygen supply to the brain. Brain edema has several subgroups, depending on its origin, i.e. cellular, vasogenic, etc. In addition, ICP may be raised by imbalance of production/absorption of cerebrospinal fluid (CSF) within the ventricular system. This may dominate the cause of raised ICP in neurological conditions, such as normal pressure hydrocephalus.

Anti-diarrheic drugs available on the market, such as lopetamide, and compounds such as lithium, bismuth, somatostatin, etc., have side effects (intestinal immobility, systemic effects). Consequently, WHO recommends (2001) strict safety testing of any drug employed for the pediatric population, in addition to oral rehydration.

Brain edema is treated with new neuroprotective regimes as well as with hypothermia, steroids, diuretics and osmotically via the blood, however, with varying degree of success. A specific inhibitor of the enzyme carbonic anhydrase, azetazolamide, inhibits CSF production by 50 %, but has not obtained a place in the clinical routine. New peptides, such as the atrial natriuretic factor, are among the candidates for a specific cure.

Consequently, there is a definite need of a new, effective drug to treat and prevent the above mentioned intestinal and neurological conditions and diseases.

The present invention relates to a molecule, termed "erucamide", and analogues, pharmaceutically acceptable salts, or solvents thereof, which have an anti-secretory effect.

Previously, the angiogenic activity (stimulation of formation of new blood vessels) of adipose tissue has been ascribed to erucamide (Wakamatsu et al., 1990). Sustained release of erucamide from a polymer matrix has been described to enhance neovascularization in regenerating skeletal muscle (Mitchell et al., 1996). Erucamide is also included in compositions for treatment of viral infections and skin membrane inflammation (WO 98/11887).

In addition, erucamide has a number of technical applications, mainly in the paper and textile industry. It is an anti-static, anti-sticking and anti-friction agent, used in dyes, inks, typing ribbons, carbon paper and packaging films, etc.

### SUMMARY OF THE INVENTION

The inventors have recently demonstrated a new, unexpected use of compounds with a formula II according to claim 1, i.e. to be effective in inhibition of hypersecretion of watery fluids, from mammalian cells. This is the first time this lipid has been shown to have antisecretory effects. The inventors have especially shown that erucamide can be used in the treatment of diseases, linked to disturbances in the systems for cellular transport of watery fluids, such as diarrhea, cerebral edema, Menière's disease, edema and glaucoma. In addition, erucamide and its analogues can be used for the treatment and/or prevention of ulcerative colitis and Crohn's disease, pregnancy associated edema (preeclampsia) and edemas of the joints.

In one aspect, the invention relates to the use of analogues of erucamide, in which one or more double bonds can be located between carbon atoms 11 and 12, 12 and 13, 13 and 14, 14 and 15 or 15 and 16, respectively, the double bond(s) may be cis- or trans-, and the carbon atom chain between carbon atoms 14 and 22 may be shortened or lengthened by 1-3 carbon atoms.

In another aspect, the invention relates to different formulations containing the active component in appropriate amounts, taking in to account that it is a water-insoluble lipid. The formulations include formulations such as tablets, capsules, elixirs, emulsions, liquids for intravenous injection, nasal spray, tablets for sublingual administration, suppositories and so on. Other useful formulations provide sustained release delivery from a suitable polymer matrix or membrane.

Formulations for intravenous injection, suppositories, sublingual tablets or nasal sprays are preferred.

The compounds of the invention are highly efficient in very low doses and have a well defined therapeutic window, thus avoiding the risk of causing potential side effects.

### DETAILED DESCRIPTION OF THE INVENTION

The present invention provides the use of a compound according to formula I 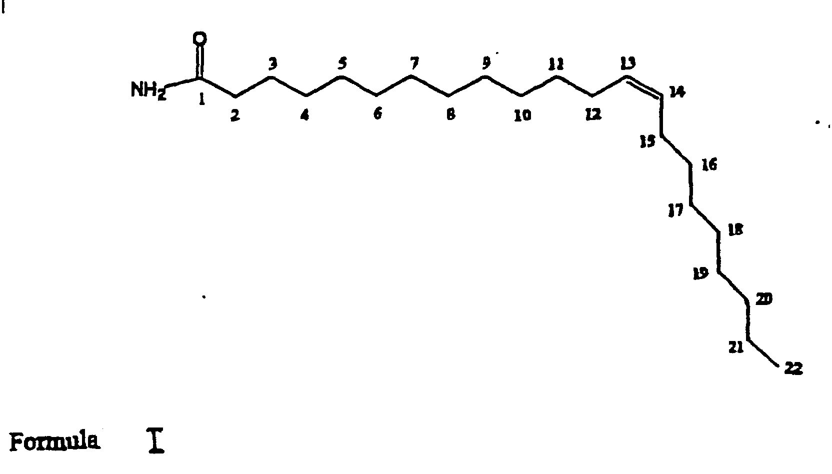 or an analogue to the compound according the above formula, in which analogue one or more cis- or trans- double bonds can be located between carbon atoms 11 and 12, 12 and 13, 13 and 14, 14 and 15 or 15 and 16, respectively, and the carbon atom chain between carbon atoms 14 and 22 may be shortened or lengthened by 1-3 carbon atoms, or a pharmaceutically acceptable salt or solvate thereof, for the preparation of a pharmaceutical composition, to be used in the treatment and/or prevention of diseases and conditions in a mammal, in which animal the Na⁺ and fluid transport are abnormal. These diseases and conditions include different forms of diarrhea, cerebral edema, pulmonary edema, glaucoma, cataract formation, pre-menstrual tension, Menière's disease, pregnancy associated edema (preeclampsia), edemas of the joints. Such conditions are exemplified by diarrhea, in which there is an abnormally increased water transport from blood to the intestine, and brain edema, in which there is an abnormally increased water transport from the blood to the brain. Assays for quantifying such increased water transport are well known in the literature (Lange 1982, Baudrie, 1990, Lorenzo, 1989).

Pharmaceutically acceptable salts include those that are formed with inorganic acids such as, for example, hydrochloric or phosphoric acids, or such organic acids as acetic, tartaric, mandelic, and the like. Salts can also be derived from inorganic bases, such as for example, sodium, potassium, ammonium, calcium or ferric hydroxides, and such organic bases as isopropylamine, trimethylamine, 2-ethylamino ethanol, histidine, procaine, and the like.

The present invention is based on experiments which aimed at ascribing an antisecretory activity to a defined chemical compound. Blood plasma from pigs was chosen as a source for the antisecretory activity, since this is easily available in the large amounts (150 L) which were necessary to provide an analyzable end product. The plasma was treated with either of two different procedures. The first starts with partly deproteinized plasma and employs the affinity of the water soluble biological activity to Isolute ENV+ (IST, Mid Glamorgan, UK). The second procedure involves extraction of the lipid fraction from plasma with organic solvents (plasma/acetone/toluene, 1:2:4, volume basis), according to Arai et al (2000). Either of these provided adequate material for the subsequent purification steps. The isolation was based on 4 steps of chromatography. The first two steps employed the affinity of the biological activity, first to Sepharose 6B (Pharmacia,), then to Isolute ENV+ (IST, Mid Glamorgan, UK). The two subsequent purification steps involved liquid chromatography with Diol (Lich Diol 10 µM, Jones Chromatography, Mid Glamorgan, UK) and C18 (Kromasil, 5 µm,Jones chromatogaphy, Mid Glamorgan, UK) columns, respectively. The final isolation step provided a single molecule. Its characterization as the previously described lipid erucamide was done with mass spectrometry (Fast atom bombardment, FAB/liquid SIMS for negative ions and gas chromatography-mass spectrometry, GC-MS). Subsequently, the antisecretory activity of erucamide was verified with the commercially available compound (Sigma/Aldrich).

The compound according to formula I is hereinafter termed "erucamide", and relates to erucylamide, cis-13-docosenoamide, cis-13-docosenoic amide, (Z)-13-docosenamide, or (Z)-docos-13-enamide and has the molecular formula: C₂₂H₄₃NO. CAS No: 112-84-5. EINECS No: 204-009-2

Throughout this specification and the claims, the words "comprises" and "comprising" are used in a non-exclusive sense.

It is to be understood that the terminology used herein is for the purpose of describing particular embodiments and aspects of the invention only.

It must be noted that, as used herein and in the appended claims, the singular forms "a", "an" and "the" include plural reference unless the context clearly dictates otherwise.

Unless defined otherwise, all technical and scientific terms used herein have the same meanings as commonly understood by one of ordinary skill in the art to which this invention belongs.

The effect asserted by erucamide is hereinafter termed "anti-secretory".

The term "CSF" means cerebro spinal fluid.

The term "subject" means any mammal, including humans. A human subject is preferred.

By medicament is meant a pharmaceutical to be used in human or veterinary medicine.

By "pharmaceutically acceptable" is meant that the carrier, diluent, excipient and salt must be compatible with the other ingredients of the formulation, and not deleterious to the recipient thereof.

An active ingredient in the formulations below, means the compounds according to the present invention and analogues, pharmaceutically acceptable salts, or solution thereof, which essentially have the above mentioned anti-secretory effects.

### Erucamide is commercially available from Sigma/Aldrich, Stockholm, Sweden

### Description of the figures:

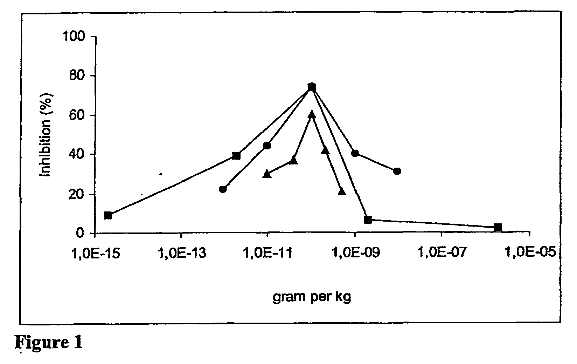

Figure 1) Dose response for the commercially available erucamide, with respect to inhibition of cholera toxin induced diarrhea in the jejunum of the rat. Maximal antisecretory activity (75% inhibition) in a 250 g rat was 1 x 10⁻¹⁰ g/kg/dose or 3 x 10⁻¹³ M. The dose response curve displayed a characteristic bell shape. Three typical experiments are shown, each comprising measurements on 5-6 rats.

A further embodiment of the invention is a pharmaceutical composition for use in the treatment or prevention of disturbances and/or defects in the fluid secretion system, comprising an effective concentration of at least one substance according to the invention, in mixture or otherwise together with at least one pharmaceutically acceptable carrier, diluent, or excipient. Examples of substances showing antisecretory activity are erucamide and the above mentioned analogues thereof. Preferably, erucamide is used.

The total amount of active ingredients in such formulations comprises from 0.1% to 99.9% by weight of the formulation.

Pharmaceutical formulations of the present invention can be prepared by procedures known in the art, using well known and readily available ingredients.

For instance, the compounds according to the invention could be incorporated with excipients, diluents or carriers and formed into tablets, capsules, powders, suspensions, and the like. Examples of excipients, diluents, and carriers suitable for such formulations include the following: fillers and extenders such as starch, sugars, mannitol, and silicic derivatives; binding agents such as carboxymethyl cellulose and other cellulose derivatives, alginates, gelatine, and polyvinyl-pyrrolidone; moisturising agents such as glycerol; disintegrating agents such as calcium carbonate and sodium bicarbonate; agents for retarding dissolution such as paraffin,; resorption accelerators such as quaternary ammonium compounds; surface active agents such as cetyl alcohol, glycerol monostearate; adsorptive carriers such as kaolin and bentonite; and lubricants such as talc, calcium and magnesium stearate, and solid polyethyl glycols.

Furthermore, the compounds can be formulated as elixirs or solutions for convenient oral administration or as solutions appropriate for parenteral administration, for example, by intramuscular, subcutaneous, intravenous or intracerebroventricular use or for topical application in the eye or the nose.

Additionally, the compounds are well suited to formulations as sustained release dosage forms and the like. The formulations can be so constituted that they release the active ingredient in a particular physiological location, possibly over time. The coatings, envelopes, and protective matrices can be made, for example, from polymeric substances or waxes.

These preparations may contain at least 5% by weight of the compounds according to the invention, the active ingredient, but could be varied depending on the subject and the disease to be treated and the special formulation used and could suitably be 5-80% by weight of the unit. The amount of the active ingredient that is contained in compositions is so high that a unit dosage form suitable for administration is obtained.

The doses required for effects of erucamide are sufficiently small, that unwanted side effects are avoided or negligible, and a typical dose of erucamide will be in the range of 10⁻¹¹-10⁻⁹ g/kg. At least 2-3 doses may be given per day. The dose is of course dependent on the type and severity of the indication being treated, age and general condition of the treated subject and on which route of administration is being chosen.

Preferred formulations of the compounds according to the invention include formulations such as tablets, capsules, elixirs, emulsions, liquids for the use as intravenous injection, nasal spray, tablets for sublingual administration, suppositories and so on. Due to the lipophilic nature of the compounds of the invention, formulations for intravenous and intracerebroventricular administration, suppositories, sublingual tablets or as nasal sprays are especially preferred.

Examples will follow that exemplifies the use of the compounds according to the present invention. The doses administered in the following examples are doses large enough to show effect of the compound, typically in the range of 10⁻¹¹-10⁻⁹ g/kg/dose.

### Example 1

A biological test system, the intestinal loop (Lange, 1982), is used to determine the antisecretory activity of erucamide and the above mentioned analogues. The test is done according to the following protocol:

Erucamide was purchased from Sigma/Aldrich, Stockholm, Sweden at a purity of > 85%, according to the manufacturer. After purification with liquid chromatography, erucamide was shown to the only compound with antisecretory activity, as verified with mass spectrometry and the biological activity test.

Rats are anaesthetized with ether. The test sample is dissolved in physiological saline containing 1% dimethyl sulfate and sonicated prior to being injected intravenously. One minute later, intestinal fluid secretion(diarrhea) is induced with 1-3 µg cholera toxin, injected into a jejunal segment, isolated with two ligatures, 100-130 mm apart. The animals recover from anaesthesia and surgery in less than 5 min. The fluid accumulation is measured by recording of length and weight of the isolated intestinal segment, obtained from rats which are sacrificed after 5 hours.

The effect of erucamide on the secretion of fluids in the gut can be seen in figure 1.

To evaluate the structure-activity relationship of erucamide (22:1 cis), other fatty acid amides and fatty acids were tested for their ability to inhibit diarrhea in the rat intestine model.

Erucic acid (cis-13-docosenoic acid, 22:1,13 cis) at 0.005 - 0.5 ng, does not display any antisecretory activity.

Arachidonic acid (20:4, 5,8,11,14 cis) displayed less than 20 % of the erucamide activity in the same concentration range.

Conclusions: Emcamide is efficient in inhibiting hypersecretion following challenge by a common cause of diarrhea. The key chemical features of erucamide that are important for its biological properties appear to be the amide functionality and the cis - double bond. This fact enables the additional use of the analogues mentioned above for the treatment of diarrhea.

### Example 2

### Effect of erucamide and analogues thereof on CSF production

Production of CSF depends mainly on active transport of Na⁺ from the blood to the brain ventricles (Davson and Segal, 1970). Water then follows the net transport of Na⁺ and other ions to maintain osmotic balance. Therefore, formation of CSF is directly proportional to Na⁺ transport into the ventricular cavities. A simple technique to measure CSF production is to determine the turnover of plasma Na⁺ into CSF. The method involves maintenance of a steady state level of ²²Na⁺ in plasma and sampling ²²Na⁺ in the CSF at various times after injection of the isotope (Knuckey et al., 1991).

Erucamide or analogues thereof, being highly potent with respect to inhibition of cholera toxin induced diarrhea in the intestine, was administered, at 5 µl/min to the right lateral ventricle via an implanted cannula in 300 g rats, anaesthetized by halothan. A 2-3 mm long micro-dialysis tubing, connecting 2 polyethylene tubings, was permanently placed in the cisterna magna for sampling of CSF. Twenty µCi ²²Na⁺, dissolved in 0.5 ml physiological saline, was given intravenously 30 min after administration of erucamide. Blood samples were taken from the tail veins at 15, 30, 90 and 180 min. Blood and CSF samples were analyzed for radioactivity. Intraventricularly administered erucamide reduced by over 40% the transport of ²²Na⁺ from plasma to CSF.

This experiment demonstrates that Na⁺, and consequently that production of CSF is reduced significantly after administration of erucamide.

### Example 3

### Menière's disease

Menières disease is characterized by vertigo, tinitus, experience of constant noise and loss of hearing. This disease probably has a heterogenous pathogenesis but the common form is thought to be due to increased pressure in the endolymph fluid inside the membrane sacs, which make up the inner ear. There is no effective treatment known for Menière's disease. An animal model for the disease was developed by Feldman and Brusilow (1976), and involves injection of cholera toxin in the inner ear to stimulate the production of endolymph fluid, in analogy with the situation in the intestine and the brain ventricles. Erucamide is administered during one month to patients with severe problems due to Menière's disease. An improvement of the symptoms shows the positive effect of the compounds of the present invention.

### Example 4

### Pulmonary edema

A rabbit model for neurogenic and/or oleic acid induced pulmonary edema is used for the determination of the effect of erucamide and analogues thereof. Erucamide is administered intravenously, 5 times/24 hours at doses ranging between 10⁻¹¹-10⁻⁹ g/kg body weight Both preventive and therapeutic aspects of the drug are investigated. A positive response in the experiment shows that emcamide or analogue thereof is effective in this treatment.

### Example 5

### Glaucoma

Ocular hypertension is induced in albino rabbits using laser injury to the aqueous outflow tissue at the anterior chamber angle. One month later, when the intraocular pressure (IOP) is increased above normal, the animals are divided into groups, which receive erucamide topically to the eye or intravenously, 5 times/24 hours at doses ranging between 10⁻¹¹ - 10⁻⁹ g/kg. The control group receive only the solvents. The IOP is recorded after one and two weeks, respectively. A positive response in the experiment shows that erucamide or analogue thereof is effective in this treatment.

### Example 6

The strong antisecretory effect of erucamide in the rat model, which employs such enterotoxins, that are deleterious to humans is the background for a clinical study. Here selected groups of patients with e.g. ulcerative colitis and Crohn's disease are treated with erucamide, administered either intravenously or according to any of the other methods described in the present application. The ratings of clinical symptoms are monitored during one month and compared with those of a placebo treated group. The results will also take in account the effects of erucamide on the inflammatory reactions of the patients, since erucamide appears to have an anti-inflammatory, as well as antisecretory effect, according to our experience. A positive response in the experiment shows that erucamide or analogue thereof is effective in this treatment.

### References;

Arai, Y., et al, Sensitive determination of anandamide in rat brain utilizing a coupled-column HPLC with fluorometric detection. Biomed. Chromatogr. 2000, 14(2):118-124.
Baudrie, V., et al, Determination of cerebrospinal fluid production rate using a push-pull perfusion procedure in the conscious rat. Fundam Clin. Pharmacol. 1990;4(3):269-274.
Davson, H., G. Hollingsworth, and M.B. Segal, *The mechanism of drainage of the cerebrospinal fluid*. Brain, 1970. 93(4): p. 665-78.
Feldman, A. and S. Brusilow, *Effects of cholera toxin on cochlear endolymph production:Model for endolymphatic hydrops*. Proc Natl Acad Sci, 1976. 73: p. 1761-1764.
Knuckey, N.W., et al., Cisterna magna microdialysis of ²²Na to evaluate ion transport and cerebrospinal fluid dynamics. J Neurosurg, 1991. 74(6): p. 965-71.
Lange S. A rat model for an in vivo assay of enterotoxic diarrhea. Microbiol Lett (1982) 15:239-242
Lorenzo, A.V., et al. Suppression of cerebrospinal fluid (CSF) production by a Na+/K+ pump inhibitor extracted from human cerebrospinal fluid. Z. Kinderchi. 1989;44 Suppl 1:24-26.
Mitchell, C.A., et al., Enhancement of neovascularization in regenerating skeletal muscle by the sustained release of erucamide from a polymer matrix. J Biomrater Appl, 1996. **10**(3): p. 230-49.
Wakamatsu, K., et al., Isolation of fatty acid amide as an angiogenic principle from bovine mesentery. Biochem Biophys Res Commun, 1990. 168(2): p. 423-9.

## Claims

1. Use of a compound according to the formula I 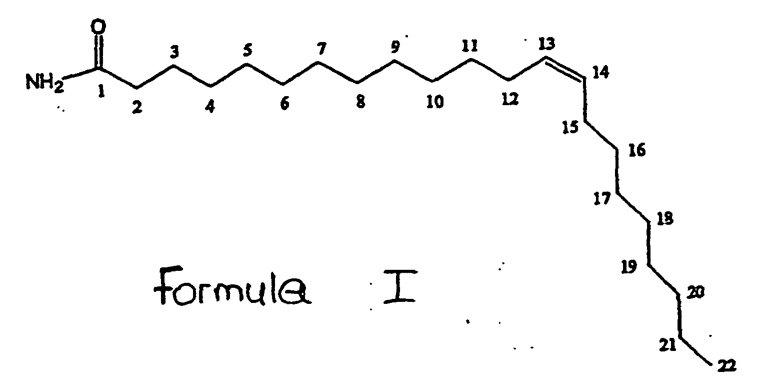 or an analogue to the compound according to the above formula, in which analogue one or more cis- or trans- double bonds can be located between carbon atoms 11 and 12, 12 and 13, 13 and 14, 14 and 15 or 15 and 16 respectively, and the carbon atom chain between carbon atoms 14 and 22 may be shortened or lengthened by 1-3 carbon atoms, or a pharmaceutically acceptable salt or solvate thereof, for the preparation of a pharmaceutical composition, to be used in the treatment of a mammal suffering from any of the conditions chosen from : diarrhea, cerebral edema, Menières disease, preeclampsia, ulcerative colitis or Crohn's disease.

2. Use according to claim 1, in which the compound is cis-13-docosenoamide.

3. Use of a medicament according to claim 1 or 2, in which the mammal is a human pregnant female suffering from preeclampsia.

4. Use according to any one of claim 1-3, in which the mammal is a human or veterinary medicine subject.

## Patentansprüche

1. Verwendung einer Verbindung gemäss Formel (I): 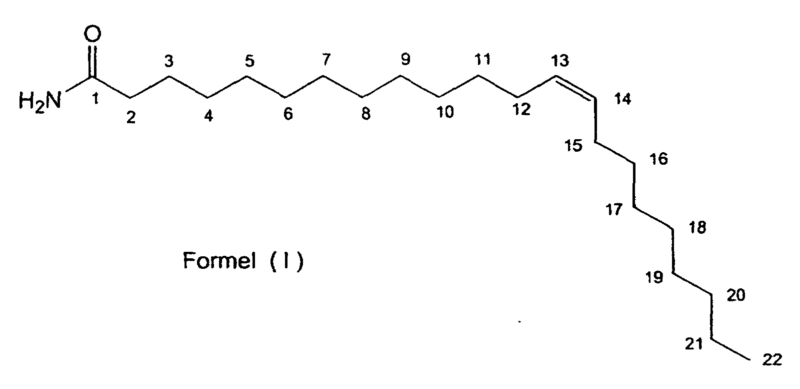 oder eines Analogs der Verbindung gemäss der obigen Formel, wobei in dem Analog eine oder mehrere cis- oder trans-Doppelbindungen zwischen den Kohlenstoffatomen 11 und 12, 12 und 13, 13 und 14, 14 und 15 bzw. 15 und 16 lokalisiert sein können, und die Kohlenstoffkette zwischen den Kohlenstoffatomen 14 und 22 kann um 1 bis 3 Kohlenstoffatome verkürzt oder verlängert sein, oder eines pharmazeutisch akzeptablen Salzes oder Solvats davon für die Herstellung einer pharmazeutischen Zusammensetzung, die zur Behandlung eines Säugers verwendet werden kann, der an einer der Bedingungen leidet, gewählt aus: Diarrhoe, Cerebralödem, Menièr'sche Erkrankung, Präeklampsie, Colitis ulcerosa oder Morbus Crohn.

2. Verwendung gemäss Anspruch 1, worin die Verbindung cis-13-Docosenoamid ist.

3. Verwendung eines Medikaments gemäss Anspruch 1 oder 2, worin der Säuger eine menschliche schwangere Frau ist, die an einer Präeklampsie leidet.

4. Verwendung gemäss einem der Ansprüche 1 bis 3, worin der Säuger ein menschliches oder veterinärmedizinisches Subjekt ist.

## Revendications

1. Emploi d'un composé de formule I : 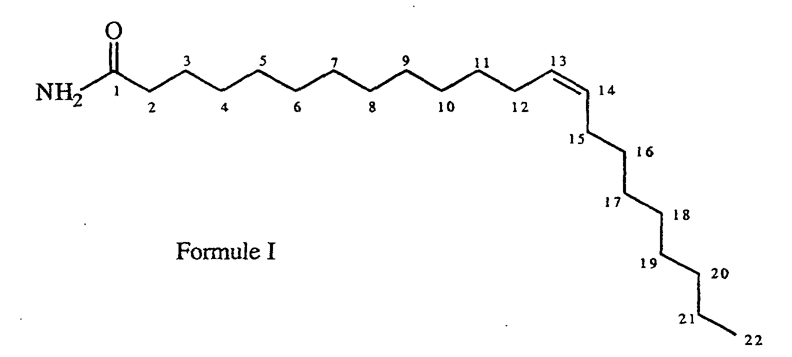 ou d'un analogue du composé de formule indiquée ci-dessus, dans lequel analogue il peut y avoir une ou plusieurs doubles liaisons, de configuration cis ou trans, respectivement situées entre les atomes de carbone 11 et 12, 12 et 13, 13 et 14, 14 et 15, ou 15 et 16, et la chaîne des atomes de carbone 14 à 22 peut être raccourcie ou rallongée d'un à trois atomes de carbone, ou d'un sel ou d'un produit d'addition de solvant d'un tel composé ou analogue, pharmacologiquement admissible, en vue de la préparation d'une composition pharmaceutique destinée à être employée pour traiter un mammifère souffrant de l'une des pathologies suivantes : diarrhée, oedème cérébral, syndrome de Ménière, prééclampsie, colite ulcéreuse, maladie de Crohn.

2. Emploi, conforme à la revendication 1, du composé cis-13-docosénamide.

3. Emploi d'un médicament, conforme à la revendication 1 ou 2, le mammifère étant une femme enceinte souffrant de prééclampsie.

4. Emploi conforme à l'une des revendications 1 à 3, le mammifère étant un patient de médecine humaine ou vétérinaire.
